(19)

Europäisches
Patentamt
European
Patent Office
Office européen
des brevets

(11)  **EP 1 775 581 A1**

(12)  **EUROPEAN PATENT APPLICATION**
published in accordance with Art. 158(3) EPC

(43) Date of publication:
**18.04.2007 Bulletin 2007/16**

(21) Application number: **05768470.6**

(22) Date of filing: **04.08.2005**

(51) Int Cl.:
*G01N 27/62* (2006.01)    *G01N 33/68* (2006.01)

(86) International application number:
**PCT/JP2005/014303**

(87) International publication number:
**WO 2006/013925 (09.02.2006 Gazette 2006/06)**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **04.08.2004 JP 2004228324**

(71) Applicant: **Eisai R&D Management Co., Ltd.
Tokyo 112-8088 (JP)**

(72) Inventors:
• **ISHIHAMA, Yasushi
Tsuruoka-shi,
Yamagata 997-0017 (JP)**
• **KAWAI, Takatoshi
c/o Tsukuba Res. Laboratories
Tsukuba-shi,
Ibaraki 3002635 (JP)**

(74) Representative: **HOFFMANN EITLE
Patent- und Rechtsanwälte
Arabellastraße 4
D-81925 München (DE)**

(54)  **EXAMINATION OF AMINO ACID SEQUENCE CONSTITUTING PEPTIDE DEPENDING ON ISOTOPIC RATIO**

(57)    It is an object of the present invention, when determining and identifying an amino acid sequence of a peptide using MS, to obtain additional information from the MS for evaluating validity of an amino acid sequence in a candidate list outputted from an identifying engine. The present invention provides a method of testing an amino acid sequence inferred by searching a peptide-related database based on peptide mass information and/or peptide modification information obtained through mass spectrometry on a peptide, the method comprising the steps: (1) calculating a theoretical value of an isotopic ratio for the peptide from the inferred amino acid sequence and/or the peptide modification information; (2) measuring a measured value of the isotopic ratio for the peptide from the peptide mass information; and (3) comparing the theoretical value and the measured value, and evaluating validity of the inferred amino acid sequence from differences between the theoretical value and the measured value.

**FIG.2A**

**FIG.2B**

MEASURED VALUES          THEORETICAL VALUES

Intensity: s=3.46, R²=0.993
Area:      s=2.78, R²=0.996

THEORETICAL VALUES

EP 1 775 581 A1

**Description**

Technical Field

The present invention relates to a test method for evaluating validity of an amino acid sequence inferred from mass spectrometry on a peptide, and more particularly relates to a test method and a test apparatus for evaluating validity of an inferred amino acid sequence by comparing theoretical values of an isotopic ratio for a peptide and measured values of the isotopic ratio for the peptide, and a program for implementing the method, and a storage medium storing the program.

Background Art

In recent years, nucleotide sequences of genes have been comprehensively analyzed, and databases of proteins and nucleic acids have been enlarged, whereby even in the case that a peptide sequence cannot be completely determined, it has become possible to search out a matching peptide sequence from a database based on partial mass spectrometry (hereinafter referred to merely as "MS") analysis information.

Broadly classifying, there are two such database search methods. One is a peptide mass fingerprinting method (PMF method; see, for example, Non-Patent Document 1: M. Mann, P. Hojrup, P. Roepstorff, Biol. Mass Spectrom., 22 (1993) 338). This is a method in which a protein is processed using a method with clear cleavage specificity such as trypsin digestion, and the masses of the resulting group of peptides are measured using MS, while proteins in a database are similarly processed *in silico,* and correlation between the measured data and the theoretical data is referred to, whereby the protein is identified. A problem with this method is that a certain number of peptides are required to distinguish the true protein from a group of proteins giving false hits. Moreover, it is generally difficult to apply the PMF method in the case of a mixture, and to increase the specificity of the search, the measured peptide masses must be highly precise. Furthermore, there is a problem that the PMF method fundamentally cannot cope with post-translational modification in which the peptide mass changes.

The other method is a method using a tandem mass spectrum. A peptide introduced into the MS is fragmented through collision induced dissociation (CID) in the MS, and partial information on the amino acid sequence of the peptide is obtained from the spectrum obtained at this time (MSMS spectrum, tandem mass spectrum, fragment spectrum, or CID spectrum), and hence identification is carried out by searching through information obtained from proteins in a database (see, for example, Non-Patent Document 2: J.K. Eng, A.L. McCormack, I. Yates, John R., Journal of the American Society for Mass Spectrometry, 5 (1994) 976, Non-Patent Document 3: M. Mann, M. Wilm, Anal. Chem., 66 (1994) 4390, Non-Patent Document 4: D.N. Perkins, D.J. Pappin, D.M. Creasy, J.S. Cottrell, Electrophoresis, 20 (1999) 3551). With this method, there is sufficient search specificity even with one peptide, and hence this method is suitable for comprehensive analysis or measurement with a mixture. Moreover, due to the high specificity, direct searching can be carried out for a genome, and this method can also cope with post-translational modification.

However, with each of the above methods, in the case that a very large number of kinds of proteins exist as in the case of mammalian tissue or cells, it is not easy to completely eliminate false hits from a protein list outputted from a search engine; even if the criteria for identification are cleverly devised, about 10 to 30% of false hit proteins are always mixed in; additional information for peptide identification is thus required.

Furthermore, even in the case of de novo sequencing in which a database is not used but rather a sequence is determined from only information obtained from an MSMS spectrum or a peptide sequencer, it is expected that additional information would play a big role in testing the validity of the determined sequence.

Disclosure of Invention

Problems to be Solved by the Invention

In view of the above circumstances, it is thus an object of the present invention, when determining and identifying an amino acid sequence of a peptide using MS, to obtain additional information from the MS for testing whether or not an amino acid sequence in a candidate list outputted from a search engine is correct.

Means for Solving the Problems

Out of additional information obtained from MS, the present inventors focused on isotopic ratios for a peptide. The present inventors had the following idea. The isotopic ratios for the elements constituting a peptide are universally constant on the earth. The composition ratios of the elements constituting the peptide can be calculated from an amino acid sequence outputted from an identifying engine, and then the isotopic ratios for the peptide can be calculated based on the isotopic ratios for the elements from the composition ratios of the elements. If the calculated isotopic ratios match the isotopic ratios actually measured by MS, then the outputted amino acid sequence can be evaluated as being correct.

In the first aspect of the present invention, there is provided a method of testing an amino acid sequence inferred by searching a peptide-related database based on peptide mass information and/or peptide modification information obtained through mass spectrometry on a peptide, the method comprising the steps:

(1) calculating a theoretical value of an isotopic ratio for the peptide from the inferred amino acid sequence and/or the peptide modification information;
(2) measuring a measured value of the isotopic ratio for the peptide from the peptide mass information; and
(3) comparing the theoretical value and the measured value, and evaluating validity of the inferred amino acid sequence from difference between the theoretical value and the measured value.

According to the preferable aspect of the testing method of the present invention, the method further comprises (4) judging whether or not the inferred amino acid sequence is correct based on evaluation of the validity, or selecting one or a plurality of amino acid sequence(s) from the inferred amino acid sequence(s) based on a value of a parameter reflecting the validity.

According to the preferable aspect of the testing method of the present invention, the selection step comprises selecting an amino acid sequence for which the parameter is not less than a predetermined value from the inferred amino acid sequence.

Further, in the second aspect of the present invention, there is provided a testing apparatus comprising a mass spectrometer and a computer having a computational unit, for testing an amino acid sequence inferred by searching a peptide-related database based on peptide mass information and/or peptide modification information obtained through mass spectrometry,

the computational unit, after receiving the peptide mass information and/or the peptide modification information, comprising:

(a) calculating means for calculating a theoretical value of an isotopic ratio for the peptide from the inferred amino acid sequence and/or the peptide modification information;
(b) measuring means for measuring a measured value of the isotopic ratio for the peptide from the peptide mass information; and
(c) evaluating means for comparing the theoretical value and the measured value, and evaluating by the computational unit validity of the inferred amino acid sequence from the difference between the theoretical value and the measured value.

According to the preferable aspect of the testing apparatus of the present invention, the computational unit further comprises (d) judgment means for judging whether or not the inferred amino acid sequence is correct based on evaluation of the validity, or further comprises calculation means for calculating a value of a parameter reflecting the validity of the inferred amino acid sequence, and wherein the amino acid sequence is selected from the inferred amino acid sequence(s) based on the parameter.

According to the preferable aspect of the testing apparatus of the present invention, the selection comprises selecting an amino acid sequence for which the parameter is not less than a predetermined value from the inferred amino acid sequence.

Furthermore, in the third aspect of the present invention, there is provided a program for causing a computer that receives peptide mass information and/or peptide modification information obtained through mass spectrometry on a peptide to test an amino acid sequence inferred by searching a peptide-related database, the program implementing the steps of:

(i) inputting the peptide mass information and/or the peptide modification information into a computational unit of the computer;
(ii) calculating by the computational unit a theoretical value of an isotopic ratio for the peptide from the inferred amino acid sequence and/or the peptide modification information;
(iii) measuring by the computational unit a measured value of the isotopic ratio for the peptide from the peptide mass information; and
(iv) comparing the theoretical value and the measured values, and evaluating by the computational unit validity of the inferred amino acid sequence from the difference between the theoretical value and the measured value.

According to the preferable aspect of the program of the present invention, the program further implements (v) judging by the computational unit whether or not the inferred amino acid sequence is correct based on evaluation of the validity, or selecting one or a plurality of amino acid sequence(s) from the inferred amino acid sequence(s) based on a value of a parameter reflecting the validity of the inferred amino acid sequence.

According to the preferable aspect of the program of the present invention, the selection comprises selecting an amino acid sequence for which the parameter is not less than a predetermined value from the inferred amino acid sequences.

In addition, in the fourth aspect of the present invention, there is provided a computer-readable storage medium storing the program according to the third aspect described above.

Note that the program or program product according to the present invention is one that causes a computer to implement the steps of the testing method according to the present invention; the program or program product can be installed or downloaded onto the computer via any of various storage media such as a CD-ROM, a magnetic disk, or a semiconductor memory.

Moreover, the term "peptide mass information" used in the present invention means information obtained through mass spectrometry, including m/z values for the peptide obtained through mass spectrometry. Furthermore, the term "peptide modification information" used in the present invention means information relating to modification carried out on the peptide in a living body or during preparation of the peptide; this does, however, also include unmodified peptide information. Examples of modification carried out in a living body include phosphorylation, saccharide chain addition, and fatty acid addition; examples of modification carried out during preparation of the peptide include enzyme digestion, reduction, and acetylation. Moreover, the term "amino acid sequence that has been inferred by searching for peptide mass information with a peptide-related database" used in the present invention means an amino acid sequence inferred through the PMF method or MSMS processing. Here, "peptide-related database" refers to a protein database or a nucleic acid database, examples being the NCBInr database as a protein database, and the GenBank database as a nucleic acid database. Moreover, the "inferred amino acid sequence" may include modified amino acids, for example amino acids that have been subjected to phosphorylation, saccharide chain addition, fatty acid addition, or the like.

Advantageous Effects of the Invention

According to the present invention, there can be provided a method for testing validity of an inferred amino acid sequence in which, when evaluating whether or not an amino acid sequence that has been inferred by carrying out a database search is correct based on amino acid sequence information or mass information obtained through MS, isotopic ratios from the MS spectrum are used as additional information.

Brief Description of Drawings

FIG. 1 illustrates a typical example of a mass spectrum of a peptide;

FIG. 2 illustrates drawings showing the measured values of the isotopic ratios from the MS spectrum of the peptide and theoretical values calculated from the inferred amino acid sequence, and the correlation between the measured values and the theoretical values in an example according to the present invention; Fig. 2 (A) illustrates the relationship for peak heights in the MS spectrum, and Fig. 2 (B) illustrates the correlation between the measured values and the theoretical values;

FIG. 3 illustrates drawings showing the measured values of isotopic ratios from the MS spectrum of the peptide and the theoretical values calculated from the inferred amino acid sequence, and the correlation between the measured values and the theoretical values in another example according to the present invention; Fig. 3 (A) illustrates the relationship for peak heights in the MS spectrum, and Fig. 3 (B) illustrates the correlation between the measured values and the theoretical values;

FIG. 4 illustrates drawings showing the measured values of isotopic ratios from the MS spectrum of the peptide and the theoretical values calculated from the inferred amino acid sequence, and the correlation between the measured values and the theoretical values in yet another example according to the present invention; Fig. 4 (A) illustrates the relationship for peak heights in the MS spectrum, and Fig. 4 (B) illustrates the correlation between the measured values and the theoretical values;

FIG. 5 illustrates a scheme of a test method according to the present invention carried out after mass spectrometry using a mass spectrometer;

FIG. 6 illustrates a functional block diagram of a test apparatus for implementing a program for the test method according to the present invention using a computer;

FIG. 7 is a flowchart showing conceptually the program for implementing the test method according to the present invention;

FIG. 8 illustrates a functional block diagram showing the detailed configuration of the computational unit used in the present invention;

FIG. 9 shows results of correlation coefficients for a group of peptides having a close mass number (2328.9-1 Da, 2328.9 Da, 2328.9+1 Da), in an example according to the present invention; and

FIG. 10 shows results of correlation coefficients for peptides having a mass number close to 939.39 ($\pm$ 1 Da), in an example according to the present invention.

Best Mode for Carrying Out the Invention

The following embodiment is merely illustrative for explaining the present invention, and the present invention is not intended to be limited thereto. The present invention can be implemented in various modes so long as there is no departure from the gist of the present invention.

Amino acid sequences tested using the present invention are amino acid sequences inferred using a method in which a database is searched from peptide mass information obtained using the PMF method (Non-Patent Document 1: M. Mann, P. Hojrup, P. Roepstorff, Biol. Mass Spectrom., 22 (1993) 338), or a method in which a database is searched from peptide amino acid sequence information obtained from a tandem mass spectrum (Non-Patent Document 2: J.K. Eng, A.L. McCormack, I. Yates, John R., Journal of the American Society for Mass Spectrometry, 5 (1994) 976, Non-Patent Document 3: M. Mann, M. Wilm, Anal. Chem., 66 (1994) 4390, Non-Patent Document 4: D.N. Perkins, D.J. Pappin, D.M. Creasy, J.S. Cottrell, Electrophoresis, 20 (1999) 3551).

Regarding the method of identifying the peptide using data obtained from MS measurement results, analysis of the obtained data and automatic identification can be carried out using commercially available software, for example Sonar MSMS (made by Genomic Solution), and a database, for example a database such as NCBInr (http://www.nc-bi.nlm.nih.gov/), IPI, or SwissProt. Inferring the amino acid sequence of the peptide using MS measurement data is easy for a person skilled in the art (see Nat. Genet., 1998: 20, 46-50; J. Cell Biol., 1998: 141, 967-977; J. Cell Biol., 2000: 148, 635-651; Nature, 2002: 415, 141-147; Nature, 2002: 415, 180-183; Curr. Opin. Cell Biol., 2003: 15, 199-205; Curr. Opin. Cell Biol., 2003: 7, 21-27).

Following is a detailed description of the method of testing inferred amino acid sequences.

1. Step of calculating isotopic ratios for peptide from inferred peptide sequence

The constituent elements of a peptide are easily calculated from the constituent elements of the amino acids. Isotopic ratios for the peptide can be calculated from the constituent elements based on the natural abundance ratios and mass numbers of stable isotopes (J.A. Yergey, Int. J. Mass Spectrom. Ion Phys., 52 (1983) 337). Using the natural abundance ratios of $^{1}H$, $^{2}H$, $^{12}C$, $^{13}C$, $^{14}N$, $^{15}N$, $^{16}O$, $^{17}O$, $^{18}O$, $^{32}S$, $^{33}S$, $^{34}S$, and $^{36}S$, calculation is carried out taking the component ratio for the first isotope peak which is for when all of the constituent elements have their lowest mass number to be the coefficient of $X°$ in the following formula 1, taking the component ratio for the second isotope peak for which one of the constituent elements is replaced with an isotope having a higher mass number to be the coefficient of $X^1$ in the following formula, and taking the component ratio for the $(n+1)^{th}$ isotope peak for which n of the constituent elements are replaced with an isotope having a higher mass number to be the coefficient of $X^n$ in the following formula. The natural abundance ratios of the elements are given, for example, in Table 3 (page 347) in J.A. Yergey, Int. J. Mass Spectrom. Ion Phys., 52 (1983) 337 (see Table 1).

Table 1

| TABLE 1 | | |
|---|---|---|
| **ELEMENT** | **MASS NUMBER** | **ISOTOPIC RATIO** |
| **C** | **12** | **0.98900** |
| | **13** | **0.01100** |
| **H** | **1** | **0.99985** |
| | **2** | **0.00015** |
| **N** | **14** | **0.99630** |
| | **15** | **0.00370** |

(continued)

| ELEMENT | MASS NUMBER | ISOTOPIC RATIO |
|---------|-------------|----------------|
| O | 16 | 0.99762 |
| | 17 | 0.00038 |
| | 18 | 0.00200 |
| | | |
| S | 32 | 0.95020 |
| | 33 | 0.00750 |
| | 34 | 0.04210 |
| | 36 | 0.00020 |

$$(P_{1H}+XP_{2H})^{N_H} \quad (P_{12C}+XP_{13C})^{N_C} \quad (P_{14N}+XP_{15N})^{N_N} \quad (P_{16O}+XP_{17O}+X^2P_{18O})^{N_O}$$

$$(P_{32S}+XP_{33S}+X^2P_{34S}+X^4P_{36S})^{N_S} \qquad \cdots\cdots\cdots \text{FORMULA 1}$$

| | | | | | |
|---|---|---|---|---|---|
| NUMBER OF H : | $N_H$ | NUMBER OF N : | $N_N$ | NUMBER OF S : | $N_S$ |
| ABUNDANCE RATIO OF $^1$H : | $P_{1H}$ | ABUNDANCE RATIO OF $^{14}$N : | $P_{14N}$ | ABUNDANCE RATIO OF $^{32}$S: | $P_{32S}$ |
| ABUNDANCE RATIO OF $^2$H : | $P_{2H}$ | ABUNDANCE RATIO OF $^{15}$N : | $P_{15N}$ | ABUNDANCE RATIO OF $^{33}$S: | $P_{33S}$ |
| NUMBER OF C : | $N_C$ | NUMBER OF O : | $N_O$ | ABUNDANCE RATIO OF $^{34}$S: | $P_{34S}$ |
| ABUNDANCE RATIO OF $^{12}$C: | $P_{12C}$ | ABUNDANCE RATIO OF $^{16}$O: | $P_{16O}$ | ABUNDANCE RATIO OF $^{36}$S: | $P_{36S}$ |
| ABUNDANCE RATIO OF $^{13}$C: | $P_{13C}$ | ABUNDANCE RATIO OF $^{17}$O: | $P_{17O}$ | | |
| | | ABUNDANCE RATIO OF $^{18}$O: | $P_{18O}$ | | |

Specifically, the component ratio for the first isotope peak and the component ratio for the second isotope peak can be calculated as follows as the coefficients of $X^0$ and $X^1$ in formula 1.

**COMPONENT RATIO FOR FIRST ISOTOPE PEAK**

$$=P_{1H}^{N_H} \quad P_{12C}^{N_C} \quad P_{14N}^{N_N} \quad P_{16O}^{N_O} \quad P_{32S}^{N_S}$$

**COMPONENT RATIO FOR SECOND ISOTOPE PEAK**

$$=N_H P_{1H}^{N_H-1} \ P_{2H} P_{12C}^{N_C} \ P_{14N}^{N_N} \ P_{16O}^{N_O} \ P_{32S}^{N_S} + N_C P_{1H}^{N_H} \ P_{12C}^{N_C-1} \ P_{13C} P_{14N}^{N_N} \ P_{16O}^{N_O} \ P_{32S}^{N_S}$$

$$+N_N P_{1H}^{N_H} \ P_{12C}^{N_C} \ P_{14N}^{N_N-1} \ P_{15N} \ P_{16O}^{N_O} \ P_{32S}^{N_S} + N_O P_{1H}^{N_H} \ P_{12C}^{N_C} \ P_{14N}^{N_N} \ P_{16O}^{N_O-1} \ P_{17O} \ P_{32S}^{N_S}$$

$$+N_S P_{1H}^{N_H} \ P_{12C}^{N_C} \ P_{14N}^{N_N} \ P_{16O}^{N_O} \ P_{32S}^{N_S-1} \ P_{33S}$$

The component ratio for the third isotope peak can similarly be calculated as the coefficient of $X^2$ in formula, and the subsequent component ratios can similarly be calculated using $X^3$, $X^4$.... Moreover, for a peptide containing other elements such as phosphorus (P), calculation can similarly be carried out by adding terms for P and any other elements to formula 1. In some cases, labeling a specified amino acid with a stable isotope is also permitted. In this case, the isotopic ratios for the peptide are calculated using, for the labeled amino acid, the isotopic abundance ratio for the labeled amino acid instead of the stable isotope natural abundance ratio. The labeling may be metabolic labeling in which a stable isotope-labeled amino acid is added to a culture solution, or may be chemical modification of the peptide with a stable isotope-labeled compound.

2. Step of measuring isotopic ratios for peptide

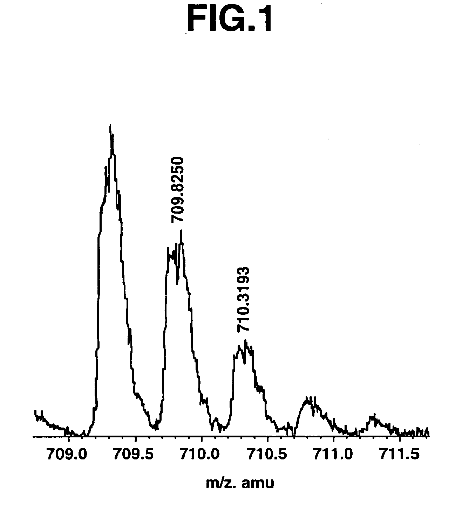

Measured values of the isotopic ratios for the peptide are measured from an MS spectrum of the peptide. A spectrum like that shown in FIG. 1 is obtained from the MS; the first peak of lowest mass is for the peptide in which all of the constituent elements have their lowest mass number, and the second peak is for the peptide in which one of the constituent elements is replaced with an isotope having a mass number one higher. The isotopic ratios can be obtained from the maximum value at each peak (the peak height) or the peak area. In some cases, an operation for removing errors from the measured values from the MS spectrum is permitted. For example, as with LCMS or the like, in the case that a plurality of spectra are obtained over time for the same peptide in accordance with the chromatography elution time, it is permitted to obtain the isotopic ratio measured values by averaging the heights or areas of corresponding peaks. Moreover, it is also possible to remove background signals by taking the differences between the peak heights (areas), and then take the ratios. Such an operation is commonly carried out when obtaining quantitative values from peaks in liquid chromatography, and it is permitted to apply such methods to the peaks in the MS spectrum.

3. Step of comparing isotopic ratio theoretical values and measured values, and evaluating validity of amino acid sequence

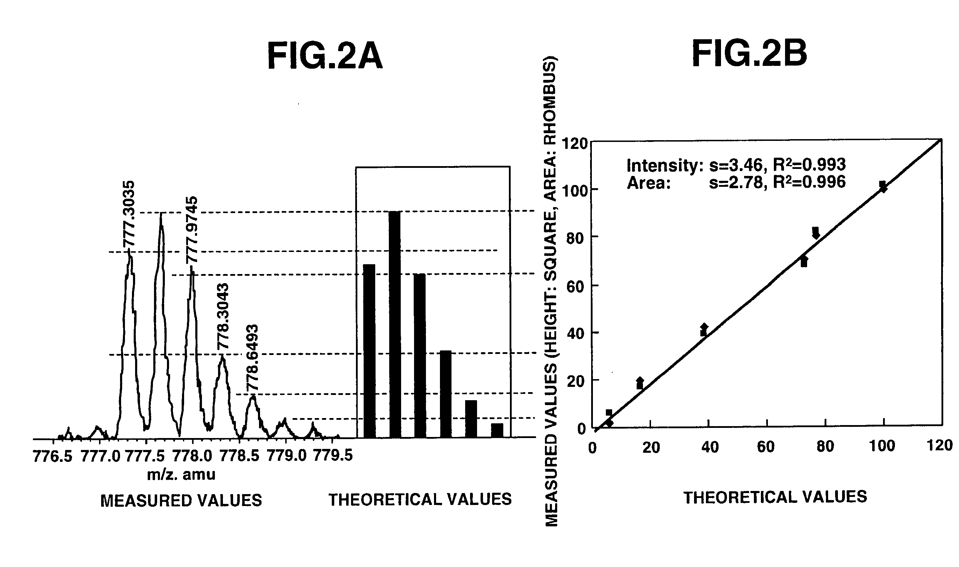

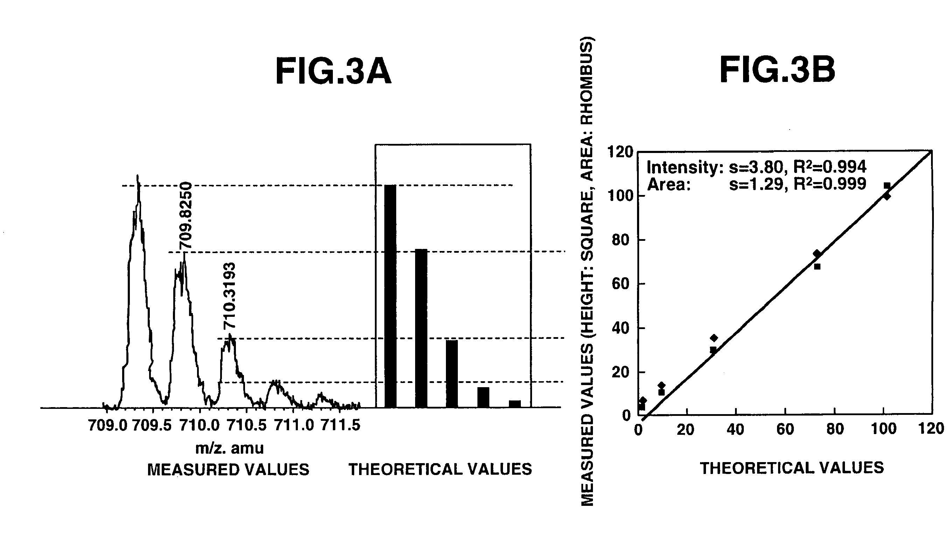

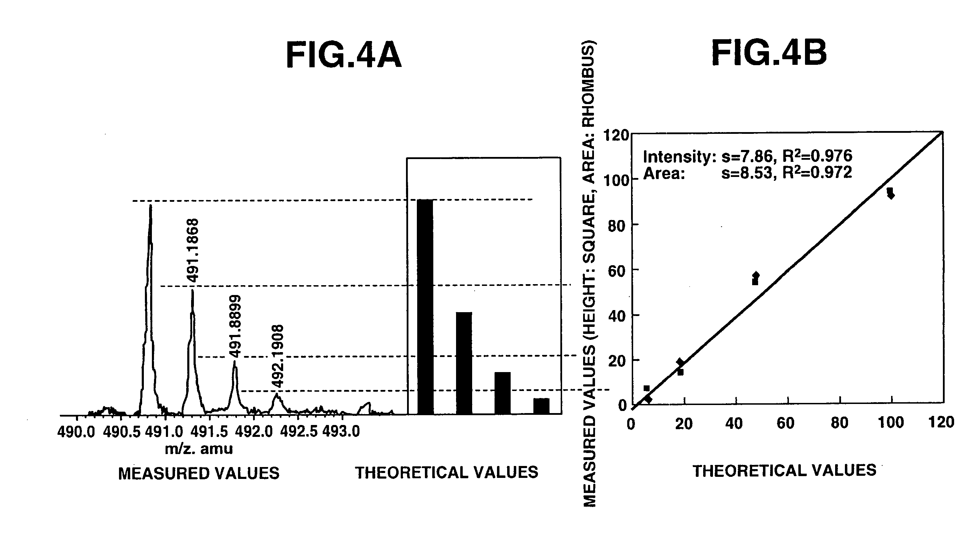

The theoretical values obtained from the above step 1. and the measured values obtained from the above step 2. are compared, so as to evaluate whether or not each inferred amino acid sequence is correct. The isotopic ratio measured values and theoretical values are normalized, and if the values are well matched with one another then it is judged that the inferred amino acid sequence is correct, whereas if the values are not well matched with one another then it is judged that the inferred amino acid sequence is wrong. Examples of the normalization method include a method of taking the ratio based on the first peak, a method of taking the ratio based on the highest peak, or a method of representing as the abundance ratio taking the whole to be 1. Moreover, it is also possible to display the normalized values on a graph, and if the values are well matched with one another judge that the inferred amino acid sequence is correct, whereas if the values are not well matched with one another judge that the inferred amino acid sequence is wrong. For example, in FIGS. 2 and 3 in the Examples described later it is judged that the sequence is correct, whereas in FIG. 4 in the Examples it is judged that the sequence is wrong.

In the present invention, in the evaluation of whether or not each inferred amino acid sequence is correct, the judgment is preferably carried out through statistical processing of the theoretical values and measured values obtained. There are no particular limitations on the statistical processing, but an example of this processing includes a method in which the measured values are subjected to linear regression relative to the theoretical values. The linear regression calculations can be carried out, for example, using the LINEST function of Microsoft Excel. If the points representing the theoretical values and measured values are close to the regression line then it is judged that the sequence is correct, whereas if these points are away from the regression line, then it is judged that the sequence is wrong. Alternatively, if the correlation coefficient between the theoretical values and the measured values is high, preferably not less than 0.98, more preferably not less than 0.99, then it is judged that the inferred amino acid sequence is correct, whereas if this correlation coefficient is low, preferably not more than 0.98, then it is judged that the inferred amino acid sequence is wrong. The statistical means is not limited to the above method; for example the test may instead be carried out using a method such as a chi-squared test of the errors between the normalized theoretical values and measured values.

The results of the test can be judged in an overall way together with an indicator of the correctness from when the amino acid sequence(s) was/were inferred, for example the threshold value for determining identification from the score from a database search engine (e.g. Mascot); in the case that there is one inferred amino acid sequence, it is evaluated whether or not this inferred amino acid sequence is valid, whereas in the case that there are a plurality of inferred amino acid sequences, it is evaluated whether or not the selection of one or a plurality of valid amino acid sequence(s) from the inferred amino acid sequences is correct. Moreover, it is also possible to carry out evaluation using the isotopic ratios for amino acid sequences in the database, and use as a parameter for inferring candidate amino acid sequences.

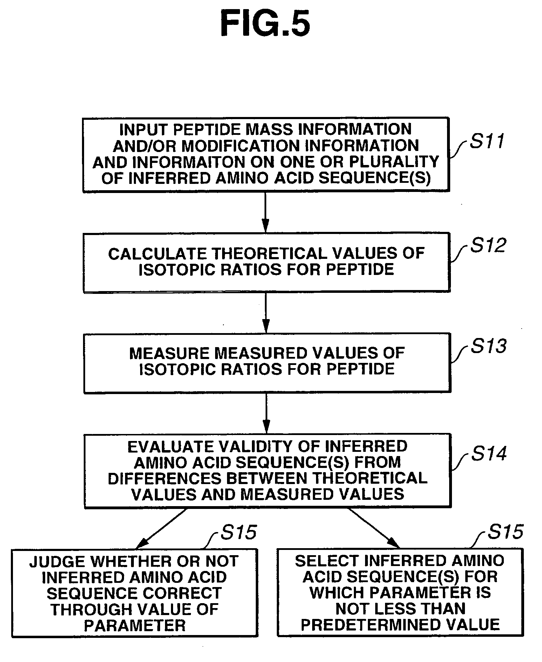

A test method according to the present invention will now be described. FIG. 5 illustrates a scheme of the test method according to the present invention carried out after mass spectrometry using the mass spectrometer. In the test method according to the present invention, first, peptide mass information and/or peptide modification information constituting the results of mass spectrometry on a peptide and one or a plurality of inferred amino acid sequence(s) are inputted (see step S11 in FIG. 5). Here, on the input side, there is an analyzer of a test apparatus according to the present invention, described later. Inferring the amino acid sequence(s) by searching any of various databases as described earlier is something easily understandable to a person skilled in the art. The constituent elements constituting the peptide and the numbers of these elements can be ascertained from the amino acid sequence(s).

Next, in step S12, based on the inferred amino acid sequence information and/or peptide modification information, in particular information on the constituent elements of the amino acids, theoretical values of the isotopic ratios for the peptide are calculated using the method of calculating the isotopic ratios for the peptide described earlier. On the other hand, in step S13, measured values of the isotopic ratios for the peptide are determined from actually measured peptide mass information.

The differences between the isotopic ratio theoretical values and measured values are evaluated from these values as described earlier (see step S14). Here, the basis for evaluating the differences can be made to be the correlation coefficient from linear regression or the parameter from a chi-squared test or the like. From the results of this evaluation, referring to a predetermined reference value, in step S15 it is judged whether or not each inferred amino acid sequence is correct. In the judgment, statistical processing can be carried out as described earlier.

Specifically, the judgment can be carried out using the value of a parameter reflecting the validity of each amino acid sequence from the results of the statistical processing, for example a correlation coefficient or a regression line correlation coefficient. In the case that there is one inferred amino acid sequence, in the case that the value of the parameter is not less than a predetermined value, it is judged that the inference is valid. On the other hand, in the case that the value of the parameter is not more than the predetermined value, it is judged that the inference is incorrect. By setting the predetermined value in advance, the evaluation / judgment of the validity of the inferred amino acid sequence can be carried out easily.

Furthermore, in the case that there are a plurality of inferred amino acid sequences, one or a plurality of amino acid sequence(s) for which the value of the parameter reflecting the validity of the amino acid sequence is not less than the predetermined value can be selected from the inferred amino acid sequences. In this way, in the case of there being one or a plurality of inferred amino acid sequence(s), the correctness of each inferred amino acid sequence can be evaluated from the value of the parameter reflecting the validity.

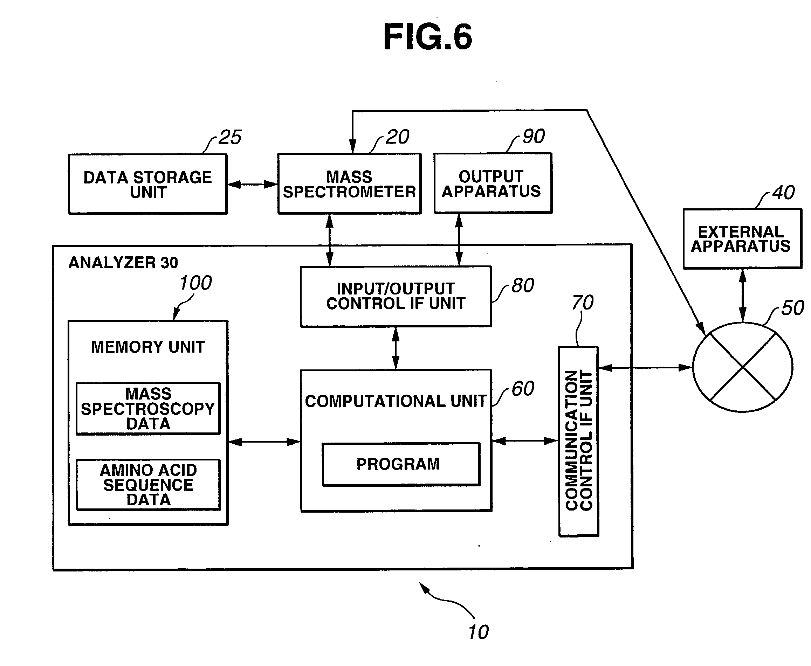

FIG. 6 illustrates the functional block diagram of the test apparatus for implementing a program for the test method according to the present invention using a computer. Note that in FIG. 6, only parts relating to the present invention are shown, this being conceptually, and these parts are constituted from a microcomputer.

Schematically, the test apparatus 10 according to the present invention comprises a mass spectrometer 20, and an analyzer 30 that processes mass spectrometry data obtained by the mass spectrometer 20. Moreover, the test apparatus 10 further comprises an external apparatus 40 that is communicably connected via a network 50 and supplies an external analysis program (not shown) for amino acid sequence determination. As shown in FIG. 6, the network 50 has a function of connecting the analyzer 30 and the external apparatus 40 together, and is for example the internet or the like. There are no particular limitations on the mass spectrometer 20 used in the present invention, which may be a commercially available mass spectrometer. The mass spectrometer 20 may itself comprise a data storage unit 25 that stores the results obtained through the measurement by the mass spectrometer 20. Moreover, the mass spectrometer 20 used in the present invention may also itself comprise a control unit for controlling the mass spectrometer 20 and an input / output unit, and furthermore may be connected to the external apparatus 40 via the network 50. The external apparatus 40 shown in FIG. 6 is connected via the network 50 to the analyzer 30, which analyzes the mass spectrometry information; the external apparatus 40 has a function of supplying a website that implements an external analysis program for homology searching or the like and an external database relating to amino acid sequence data or the like for a user.

Here, the external apparatus 40 may be constituted as a web server, an ASP server, or the like, and the hardware thereof may be constituted from a generally commercially available information processing apparatus such as a workstation or a personal computer and peripherals. The various functions of the external apparatus 40 are realized by a CPU, a disk drive, a memory, input devices, output devices, a communication controller and so on in the hardware configuration of the external apparatus, programs for controlling the above, and so on. In the present invention, a database such as NCBInr can be used for the external apparatus.

Schematically, the analyzer 30 shown in FIG. 6 has a computational unit 60 such as a CPU that carries out overall control of the mass spectrometer 20, a communication control interface unit 70 that is connected to a communication apparatus (not shown) such as a router connected to a communication line or the like, an input / output control interface unit 80 connected to the mass spectrometer 20 and an output apparatus 90 such as a display or a printer, and a memory unit 100 that stores various databases. The respective units are connected together communicably via communication channels as required. Furthermore, the analyzer 30 in the present invention is connected communicably to the network via the communication apparatus such as a router and a wired or wireless communication line such as a private line. The various databases (mass spectrometry data, amino acid sequence data, etc.) stored in the memory unit 100 are on storage means such as a fixed disk drive, the storage means storing files, data and so on. Of the component elements of the memory unit 100, the mass spectrometry information is, for example, peptide mass information obtained by the mass spectrometer 20. Moreover, the amino acid sequence data may be amino acid sequence data comprised of the results of analyzing mass spectra obtained by the mass spectrometer, or external amino acid sequence data that can be accessed via the internet. Furthermore, the data may also be in-house data created by copying databases as above or storing original sequence information and further assigning original identification numbers.

The computational unit 60 is an apparatus that stores a program for implementing the analytical method according to the present invention, and controls the analyzer 30, and thus the whole of the test apparatus 10. The computational unit 60 has a control program such as an OS (operating system), programs stipulating various processing procedures and so on, and an internal memory (not shown) for storing required data, and carries out data processing for implementing the various processing using these programs and so on. Note that the program for implementing the test method according to the present invention may also be stored in the memory unit 100.

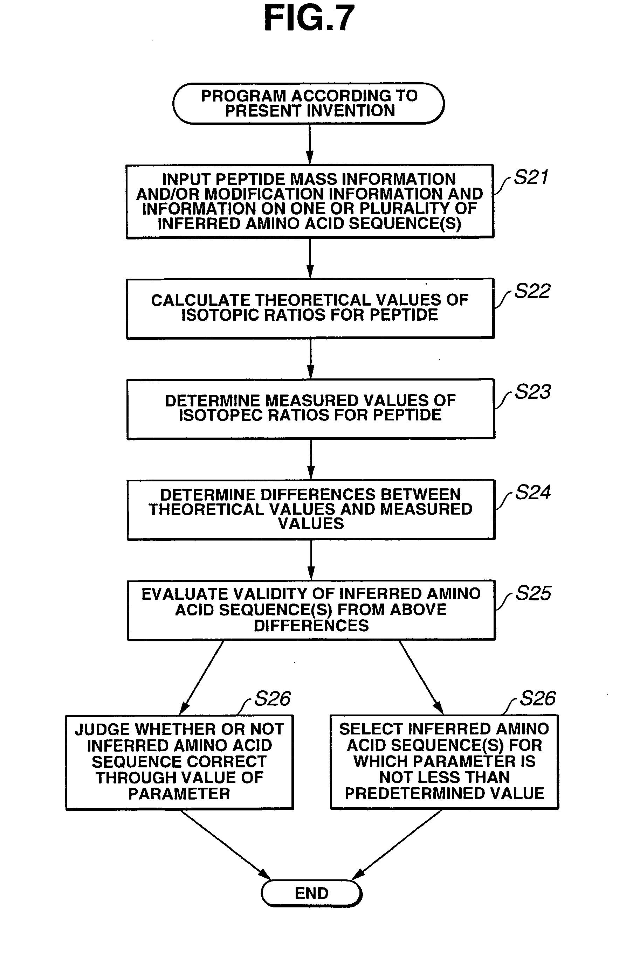

FIG. 7 illustrates a flowchart showing conceptually the program for implementing the test method according to the present invention. In step S21, the computational unit 60 acquires peptide mass information and/or modification information obtained by the mass spectrometer 20, or information relating to one or a plurality of amino acid sequence(s) inferred by searching for this information with a peptide-related database, for example amino acid sequence(s) inferred through MS/MS processing while comparing with an external database, for example the NCBInr database, via the Internet 50 through the communication control interface unit. The acquired mass spectrometry data is then stored in the memory unit 100 as required, and at this time, to facilitate data searching for the convenience of the analysis, described below, identification numbers such as scan numbers may be assigned to the mass spectrometry data. On the other hand, after the computational unit 60 of the test apparatus 10 according to the present invention has acquired the peptide mass information and/or modification information obtained by the mass spectrometer 20, information relating to amino acid sequence(s) inferred through MS/MS processing may be acquired by the computational unit 60 while comparing with an external database.

As shown in step S22, isotopic ratio theoretical values for the peptide in question are calculated from the acquired inferred amino acid sequences and/or peptide modification information. For these theoretical values, the constituent elements of the peptide are determined from the amino acid sequence of the peptide, and the theoretical values are calculated from the stable isotope natural abundance ratios and mass numbers for the constituent elements. On the other hand, in step S23, the actually measured isotopic ratio measured values for the peptide are determined from the peptide mass information.

Next, in step S24, the differences between the theoretical values and the measured values are determined, and the validity of each inferred amino acid sequence for the peptide is evaluated from the differences (see step S25). In this evaluation of the validity, the judgment is preferably carried out by carrying out statistical processing on the theoretical values and measured values obtained. Example of the statistical processing includes a method in which the measured values are subjected to linear regression relative to the theoretical values. In the case that there is one inferred amino acid sequence, if the theoretical values approximately match the measured values, for example if the value of the linear regression correlation coefficient, which is a parameter reflecting the validity, is not less than 0.98, more preferably not less than 0.99, then it is ascertained that the inferred amino acid sequence is correct.

On the other hand, in the case that there are a plurality of inferred amino acid sequences, the judgment of the inferred amino acid sequence correctness can be carried out by selecting from the inferred amino acid 0sequences one or a plurality of amino acid sequence(s) for which the value of the above parameter is not less than a predetermined value.

The judgment of the inferred amino acid sequence correctness can be carried out by selecting from the inferred amino acid sequences zero, one or a plurality of amino acid sequence(s) for which the value of the parameter reflecting the validity, preferably the linear regression correlation coefficient, is at least a desired value. Here, zero means that the inferred amino acid sequences did not include any amino acid sequences judged to be correct. Moreover, in the case that the parameter is the linear regression correlation coefficient, the desired value can be set to be a value of not less than 0.98, preferably not less than 0.99.

Then, data such as the value of the parameter obtained through the analysis by the computational unit can be displayed or printed by the output apparatus 90 such as the display or printer as required.

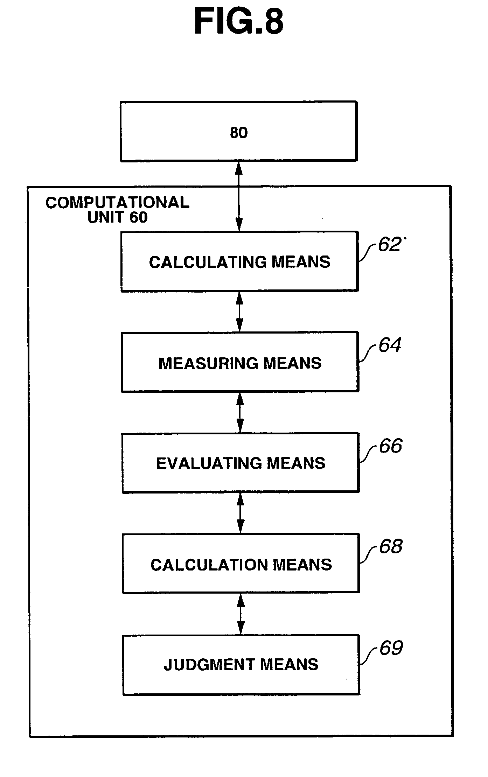

FIG. 8 illustrates a functional block diagram showing the detailed configuration of the computational unit 60 used in the present invention. As described above, for implementing the test method according to the present invention described with reference to FIG. 7, the computational unit 60 receives via the input / output control interface unit 80 peptide mass information obtained by the mass spectrometer 20. In the present invention, the computational unit 60 comprises calculating means 62, measuring means 64, evaluating means 66, judgment means 69, and calculation means 68. The calculating means 62 calculates the isotopic ratio theoretical values for the peptide from the amino acid sequence(s) inferred using an external database and/or the peptide modification information. On the other hand, the measuring means 64 measures the isotopic ratio measured values for the peptide from the peptide mass information from the mass spectrometer 20. Based on the isotopic ratio theoretical values and measured values obtained by the calculating means 62 and the measuring means 64, the evaluating means 66 then determines the differences between the theoretical values and the measured values. The evaluating means 66 determines the differences between the theoretical values and the measured values. This difference can also be determined using a parameter reflecting the validity of the inferred amino acid sequence. The evaluating means 66 has the calculation means 68 which calculates the value of this parameter, and then evaluates the validity_of the inferred amino acid sequence.

Furthermore, based on the results from the calculation means 68, the judgment means 69 judges whether or not each inferred amino acid sequence is correct. Here, from the value of the differences between the isotopic ratio measured values and theoretical values, in the case that there is no statistically significant difference, it is judged that the inferred amino acid sequence is correct, whereas in the case that there is a statistically significant difference, it is judged that the inferred amino acid sequence is incorrect. Specifically, from the value of the parameter that is the result from the calculation means 68, in the case that difference is observed between the isotopic ratio theoretical values and measured values, for example in the case that, as the predetermined value of the parameter, the value of the linear regression correlation coefficient is less than 0.98, it can be judged that the inferred amino acid sequence is incorrect.

The computational unit 60 used in the present invention has been described as being disposed in the analyzer 30, but as required the test method according to the present invention can also be implemented if the computational unit 60 is disposed in the mass spectrometer 20 instead.

Examples

The present invention will now be described in more detail through the following examples. However, the scope of the present invention is not limited thereto. Various modifications could be made by a person skilled in the art based on the description of the present invention, and such modifications are included in the present invention.

The following gives specific examples in which a database search was carried out based on amino acid sequence information obtained from MS, and inferred amino acid sequences were tested using isotope abundance ratios.

As a sample, the whole brain of a mouse was removed, and stored by freezing. The sample was homogenized using a Teflon® homogenizer, and undamaged cells, nuclei and so on were removed by centrifuging for 5 minutes at 500xg. Next, the supernatant was centrifuged for 1 hour at $100,000 \times g$ so as to prepare a soluble fraction. The protein mass was measured to be 3.12 mg / mL. The soluble fraction was taken as a fractionated sample.

Next, the following operation was carried out for 2 ml (2 tubes each of 1 mL) of each fractionated sample. 500 $\mu$L of a 0.5 M Tris buffer solution (pH 8.3, made by Sigma) to which urea (Bio-Rad Cat. No. 161-0731) had been added to 8 M and 3 mg of dithiothreitol (Wako Pure Chemical Industries Cat. No. 045-08974: DTT) had been added per 1 mL was added to each fractionated sample, and incubation was carried out for 3 hours at 37°C so as to reduce cysteine residues in the proteins. After that, 500 $\mu$L of a 0.5 M Tris buffer solution (pH 8.3) to which urea had been added to 8 M and 8 mg of acrylamide (Bio-Rad Cat. No. 161-0107) had been added was added to each fractionated sample, and incubation was carried out for 3 hours at room temperature so as to alkylate the cysteine residues. 8 mg of DTT was then added so as to deactivate excess acrylamide. Using a Snakeskin (Pierce Cat. No. 68100) dialysis tube with a cutoff at a molecular weight of 10,000, the reduction / alkylation reagents were removed by carrying out dialysis for 24 hours at 4°C using a 10 mM ammonium hydrogen carbonate buffer solution in an amount of 1000 times, and then the fractionated sample was freeze-dried using Speedvac.

Each fractionated sample was redissolved in 200 $\mu$L of a 0.2% octyl $\beta$-glucoside aqueous solution containing 8 M of urea, and dilution was carried out by a factor of 5 with 50 mM ammonium hydrogencarbonate, making up to a total of 1 mL. 100 $\mu$L of trypsin (Promega Cat. No. V5111) was added per 0.3 mg of proteins, and digestion was carried out for 24 hours at 37°C. 50 $\mu$L of ammonia water and 0.5 mL of ultra-pure water were added to the digested sample, centrifuging was carried out for 1 minute at 20,000 G, and the supernatant was injected into an anion exchange column (Mini-Q PC 3.2/3: Amersham Biosciences Cat. No. 17-0686-01). The HPLC conditions were made to be a flow rate of 0.2 mL/min, and UV detection wavelengths of 235 nm and 280 nm. Mobile phase A was made to be 25 mM ammonia with 5% acetonitrile, and mobile phase B was made to be 1 M ammonium acetate with 5% acetonitrile at pH 8.6; regarding the gradient, 100% mobile phase A was used for the first 5 minutes, the mobile phase concentration was increased over the next 40 minutes linearly up to 40%, mobile phase B was made to be 100% for the next 15 minutes, and then flushing was carried out for 5 minutes. Division into fractions every 1 minute was carried out, and the fractions eluted out from the column were made acidic by adding TFA. The fractions from 27 minutes to 30 minutes were selected as samples, and each of these was washed with acetonitrile in advance, and then applied into a StageTip C18 (made in-house, J. Rappsilber, Y Ishihama, M. Mann, Anal. Chem., 75 (2003) 663) that had been conditioned with 0.1 % TFA water in advance, and then washed 3 times with 20 $\mu$L of 0.1 % TFA water containing 5% acetonitrile, and desalinated by eluting with 5 $\mu$L of 0.1 % TFA water containing 70% acetonitrile. The solvent was evaporated off using a Speedvac, and then the sample was redissolved in 5 $\mu$L of 0.1 % TFA water containing 5% acetonitrile.

Next, each of the samples separated through the HPLC was subjected to measurement by LC (C18 column) / MS (Applied Biosystems / MDS-Sciex QSTAR Pulsar i). Regarding the conditions at this time, on the HPLC side, 0.5% acetic acid water as mobile phase A, and 0.5% acetic acid water containing 80% acetonitrile as mobile phase B were used with a 0.1 $\times$ 150 mm electrospray integrated column made in-house (Y. Ishihama, J. Rappsilber, J.S. Andersen, M. Mann, J. Chromatogr. A, 979 (2002) 233) packed with C18 silica gel (ReproSil-Pur 120 C18-AQ, 3μm), the initial B concentration was made to be 5%, mobile phase B was increased linearly to 10% over the first 5 minutes, linearly to 30% over the next 60 minutes, and then linearly to 100% over the next 5 minutes, then mobile phase B was held at 100% for 10 minutes, and then mobile phase B was made to be 5%, and after 30 minutes the next sample was injected in. Regarding the apparatus, an LC-10A series ROM made by Shimadzu was made to be micro-compatible, and as the mixing chamber, the attached one made by Shimadzu was removed, and a T connector made by Valco was used. For the flow rate, a flow splitting system was used, and adjustment was carried out such that the flow rate in the column was approximately 200 to 400 nL/min. 3 μL of each sample was injected in using a PAL autosampler made by CTC; after first being injected into the sample loop of the injector, the sample was fed into the analysis column. A column holder specially ordered from Nikkyo Technos was attached to the QSTAR Pulsar i made by Applied Biosystems / MDS-Sciex which was equipped with an XYZ stage made by Protana, so that the position of the electrospray integrated column could be freely adjusted. An ESI voltage of 2.4 KV was applied through a metal connector made by Valco on the pump side of the column. Regarding the measurement, in information dependent acquisition mode, a survey scan was carried out for 1 second, and then a maximum of four MSMS scans (each 1.5 seconds) were carried out. Switching from the MSMS mode to the survey scan was made to be every one spectrum.

For the data obtained, automatic protein identification was carried out using Mascot (Matrix science) and the NCBInr database. Out of the outputted results, the three peptides shown in Table 2 were selected, and test was carried out using isotopic ratios.

Table 2

### TABLE 2

| NO. | INFERRED AMINO ACID SEQUENCE | ORIGINATING PROTEIN | MASCOT SCORE | OBSERVED m/z | PEPTIDE MASS |
|---|---|---|---|---|---|
| 1 | AFVHWYVGEGMEEGEFSEAR | tubulin alpha | 63 | 777.3087 | 2329.0109 |
| 2 | ILDSVGIEADDDR | ribosomal protein, large P2 | 93 | 709.3246 | 1416.6732 |
| 3 | MAAGQEDDK+OXIDATION (M) | similar to hypothetical protein MGC35338 | 22 | 490.6823 | 979.3916 |

The threshold value of the Mascot score for determining identification (95%) is 37, and hence it is thought that no. 1 and 2 peptides were identified correctly, whereas no. 3 peptide was not identified correctly. From the molecular formulae of these three peptides, the theoretical values of the isotopic ratios were calculated using an accessory function (Tools/Calculators/Isotope Distribution) of Analyst QS (Applied Biosystems / MDS-Sciex) which is measurement software for QSTAR. Moreover, for the measured values of the isotopic ratios, the peak height (intensity) and area were determined for each isotope using the peak integration function of Analyst QS, and the measured values were compared with the theoretical values. The results for no. 1 to 3 peptides are shown in FIGS. 2 to 4 respectively.

For no. 1 and 2 peptides which were thought from the Mascot score to have been identified correctly, the theoretical values and measured values of the isotopic ratios agreed well with one another, whereas for no. 3 peptide which was thought to have not been identified correctly, there was found to be difference between the theoretical values and the measured values.

The theoretical values and measured values of the isotopic ratios (peak heights and peak areas) were subjected to linear regression using the LINEST function of Microsoft Excel. For no. 1 and 2 peptides which were thought to have been identified correctly, good correlation was exhibited with the correlation coefficient ($R^2$) being greater than 0.99, whereas for no. 3 peptide which was thought to have not been identified correctly, the correlation coefficient was 0.97; it is thus clear that the validity of the inferred amino acid sequence can be tested by determining the correlation between the measured values and the theoretical values.

Next, an example for a case of using actual isotopic ratio measured values, comparing with the isotopic ratios for all of the peptides in a database, and selecting a candidate peptide group will be described. For the case that the spectrum of FIG. 2 was obtained, the actual isotopic ratio measured values were compared with the isotopic ratios for all of the peptides in a database having the molecular weight in question, and a candidate peptide group was selected.

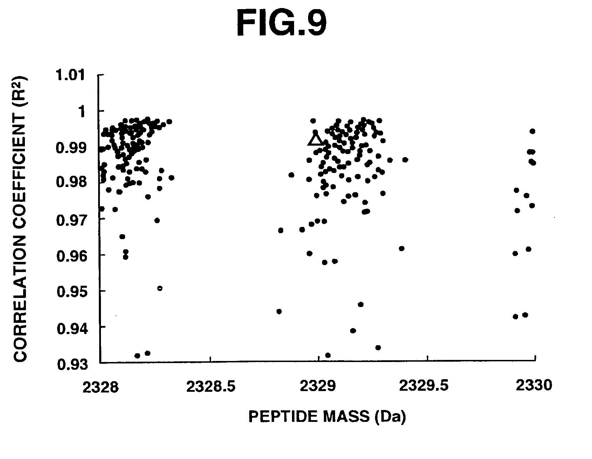

From the difference in m/z between the isotope peaks for the peptide in the spectrum of FIG. 2, the charge is 3, and hence the actual measured value of the mass number of the peptide is 2328.9. Trypsin digestion was carried out *in silico* using mouse proteins (40,981 proteins) in the July 1, 2004 version of the International Protein Index (IPI) database (ftp: //ftp.ebi.ac.uk/pub/databases/IPI/current/ipi.MOUSE.fasta.gz), and of the obtained peptides, there were 753,926 ones having a unique sequence for at least five residues. The isotopic ratios (actual measured values) obtained from FIG. 2 were subjected to regression against the isotopic ratios (theoretical values) for the peptides in this group, and the correlation coefficient for the regression line was calculated. FIG. 9 shows the results for the group of peptides having a close mass number (2328.9-1 Da, 2328.9 Da, 2328.9+1 Da).

The criterion for the correlation coefficient varies depending on the measurement apparatus and conditions, but in the case of taking the criterion to be the coefficient, which is a parameter reflecting the validity of the inferred amino acid sequence, being not less than 0.99, the 360 candidate peptides selected from the mass number were narrowed down using the isotopic ratios to 160. It was ascertained that the candidate sequence selected as the correct sequence using Mascot indicated by "Δ" in FIG. 9 was included in the sequences obtained through the narrowing down.

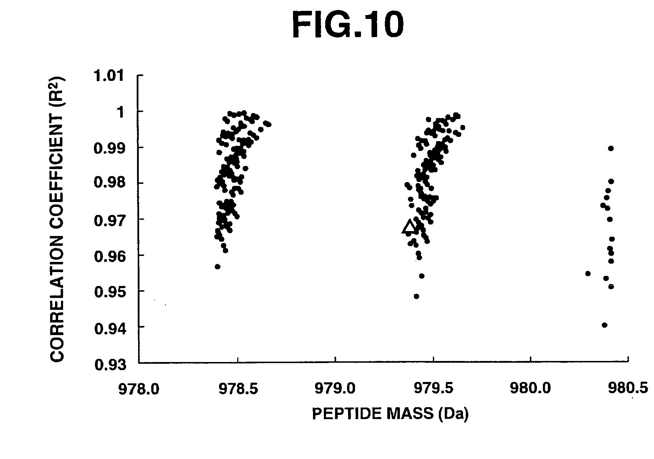

Similarly, for the case that the spectrum of FIG. 4 was obtained, the actual measured isotopic ratio values were subjected to regression analysis against the theoretical isotopic ratios for the 753,926 peptides as above, and the correlation coefficient for the regression line was examined. FIG. 10 shows the data for the peptides having a mass number close to 939.39 ($\pm$ 1 Da). The candidate sequence according to Mascot is indicated by "Δ" in FIG. 10. In the case that the criterion for the correlation coefficient was made to be not less than 0.99, the 1203 candidate peptides could be narrowed down to 362, and it was possible to eliminate from the candidate peptide group the sequence that was considered to be wrong according to Mascot (see FIG. 10).

Based on data saying that a peptide is one produced through trypsin digestion, it was possible to narrow down the candidate peptides from the isotopic ratios, i.e. select a plurality of amino acid sequences from the inferred amino acid sequences; there was no contradiction between the sequences obtained through the narrowing down and the determination of correctness according to Mascot. It is thus thought that isotopic ratios can be used as novel parameters in the narrowing down of candidate peptides.

Industrial Applicability

According to the present invention, when identifying a peptide in proteome art, when evaluating whether or not an amino acid sequence that has been inferred by carrying out a database search is correct based on amino acid sequence information or mass information obtained through MS, isotopic ratios from the MS spectrum can be used as additional information, whereby the peptide identification can be carried out with higher precision.

**Claims**

1. A method of testing an amino acid sequence inferred by searching a peptide-related database based on peptide mass information and/or peptide modification information obtained through mass spectrometry on a peptide, the method comprising the steps:

   (1) calculating a theoretical value of an isotopic ratio for the peptide from the inferred amino acid sequence and/or the peptide modification information;
   (2) measuring a measured value of the isotopic ratio for the peptide from the peptide mass information; and
   (3) comparing the theoretical value and the measured value, and evaluating validity of the inferred amino acid sequence from difference between the theoretical value and the measured value.

2. The method of testing according to Claim 1, further comprising (4) judging whether or not the inferred amino acid sequence is correct based on evaluation of the validity, or selecting one or a plurality of amino acid sequence(s) from the inferred amino acid sequence(s) based on a value of a parameter reflecting the validity.

3. The method of testing according to Claim 2, wherein the selection step comprises selecting an amino acid sequence for which the parameter is not less than a predetermined value from the inferred amino acid sequence.

4. An testing apparatus comprising a mass spectrometer and a computer having a computational unit, for testing an amino acid sequence inferred by searching a peptide-related database based on peptide mass information and/or peptide modification information obtained through mass spectrometry,
the computational unit, after receiving the peptide mass information and/or the peptide modification information, comprising:

(a) calculating means for calculating a theoretical value of an isotopic ratio for the peptide from the inferred amino acid sequence and/or the peptide modification information;

(b) measuring means for measuring a measured value of the isotopic ratio for the peptide from the peptide mass information; and

(c) evaluating means for comparing the theoretical value and the measured value, and evaluating by the computational unit validity of the inferred amino acid sequence from the difference between the theoretical value and the measured value.

5. The testing apparatus according to Claim 4, wherein the computational unit further comprises (d) judgment means for judging whether or not the inferred amino acid sequence is correct based on evaluation of the validity, or further comprises calculation means for calculating a value of a parameter reflecting the validity of the inferred amino acid sequence, and wherein the amino acid sequence is selected from the inferred amino acid sequence(s) based on the parameter.

6. The testing apparatus according to Claim 5, wherein the selection comprises selecting an amino acid sequence for which the parameter is not less than a predetermined value from the inferred amino acid sequence.

7. A program for causing a computer that receives peptide mass information and/or peptide modification information obtained through mass spectrometry on a peptide to test an amino acid sequence inferred by searching a peptide-related database, the program implementing the steps of:

(i) inputting the peptide mass information and/or the peptide modification information into a computational unit of the computer;

(ii) calculating by the computational unit a theoretical value of an isotopic ratio for the peptide from the inferred amino acid sequence and/or the peptide modification information;

(iii) measuring by the computational unit a measured value of the isotopic ratio for the peptide from the peptide mass information; and

(iv) comparing the theoretical value and the measured values, and evaluating by the computational unit validity of the inferred amino acid sequence from the difference between the theoretical value and the measured value.

8. The program according to Claim 7, further comprising (v) judging by the computational unit whether or not the inferred amino acid sequence is correct based on evaluation of the validity, or selecting one or a plurality of amino acid sequence(s) from the inferred amino acid sequence(s) based on a value of a parameter reflecting the validity of the inferred amino acid sequence.

9. The program according to Claim 8, wherein the selection comprises selecting an amino acid sequence for which the parameter is not less than a predetermined value from the inferred amino acid sequences.

10. A computer-readable storage medium storing the program according to any one of Claims 7 to 9.

# FIG.1

# FIG.2B

Intensity: s=3.46, R²=0.993
Area: s=2.78, R²=0.996

MEASURED VALUES (HEIGHT: SQUARE, AREA: RHOMBUS)

THEORETICAL VALUES

# FIG.2A

THEORETICAL VALUES

777.3035
777.9745
778.3043
778.6493

776.5 777.0 777.5 778.0 778.5 779.0 779.5
m/z. amu

MEASURED VALUES

15

# FIG.3A

709.8250

710.3193

709.0 709.5 710.0 710.5 711.0 711.5

m/z. amu

MEASURED VALUES        THEORETICAL VALUES

# FIG.3B

Intensity: s=3.80, R²=0.994
Area:       s=1.29, R²=0.999

MEASURED VALUES (HEIGHT: SQUARE, AREA: RHOMBUS)

THEORETICAL VALUES

EP 1 775 581 A1

FIG.4B

FIG.4A

17

# FIG.5

```
INPUT PEPTIDE MASS INFORMATION
AND/OR MODIFICATION INFORMATION          ⌐S11
AND INFORMAITON ON ONE OR PLURALITY
OF INFERRED AMINO ACID SEQUENCE(S)
```

```
CALCULATE THEORETICAL VALUES OF          ⌐S12
ISOTOPIC RATIOS FOR PEPTIDE
```

```
MEASURE MEASURED VALUES OF               ⌐S13
ISOTOPIC RATIOS FOR PEPTIDE
```

```
EVALUATE VALIDITY OF INFERRED
AMINO ACID SEQUENCE(S) FROM              ⌐S14
DIFFERENCES BETWEEN THEORETICAL
VALUES AND MEASURED VALUES
```

```
          ⌐S15                                    ⌐S15
JUDGE WHETHER OR NOT              SELECT INFERRED AMINO
INFERRED AMINO ACID              ACID SEQUENCE(S) FOR
SEQUENCE CORRECT                 WHICH PARAMETER IS
THROUGH VALUE OF                 NOT LESS THAN
PARAMETER                        PREDETERMINED VALUE
```

# FIG.6

**ANALYZER 30**

DATA STORAGE UNIT — 25
MASS SPECTROMETER — 20
OUTPUT APPARATUS — 90
EXTERNAL APPARATUS — 40
MEMORY UNIT — 100
MASS SPECTROSCOPY DATA
AMINO ACID SEQUENCE DATA
INPUT/OUTPUT CONTROL IF UNIT — 80
COMPUTATIONAL UNIT — 60
PROGRAM
COMMUNICATION CONTROL IF UNIT — 70
50
10

EP 1 775 581 A1

# FIG.7

PROGRAM ACCORDING TO
PRESENT INVENTION

↓

INPUT PEPTIDE MASS INFORMATION
AND/OR MODIFICATION INFORMATION AND
INFORMATION ON ONE OR PLURALITY OF
INFERRED AMINO ACID SEQUENCE(S) — S21

↓

CALCULATE THEORETICAL VALUES OF
ISOTOPIC RATIOS FOR PEPTIDE — S22

↓

DETERMINE MEASURED VALUES OF
ISOTOPEC RATIOS FOR PEPTIDE — S23

↓

DETERMINE DIFFERENCES BETWEEN
THEORETICAL VALUES AND MEASURED
VALUES — S24

↓

EVALUATE VALIDITY OF INFERRED AMINO
ACID SEQUENCE(S) FROM ABOVE
DIFFERENCES — S25

JUDGE WHETHER OR NOT
INFERRED AMINO ACID
SEQUENCE CORRECT
THROUGH VALUE OF
PARAMETER — S26

SELECT INFERRED AMINO
ACID SEQUENCE(S) FOR
WHICH PARAMETER IS
NOT LESS THAN
PREDETERMINED VALUE — S26

END

20

# FIG.8

```
┌─────────────────────────────────────┐
│                 80                   │
└─────────────────────────────────────┘
              ↕
┌──────────────────────────────────────────────┐
│ COMPUTATIONAL                                  │
│ UNIT 60                                        │
│        ┌─────────────────────────┐             │
│        │   CALCULATING MEANS     │  ⌐62·       │
│        └─────────────────────────┘             │
│                  ↕                             │
│        ┌─────────────────────────┐             │
│        │    MEASURING MEANS      │  ⌐64        │
│        └─────────────────────────┘             │
│                  ↕                             │
│        ┌─────────────────────────┐             │
│        │   EVALUATING MEANS      │  ⌐66        │
│        └─────────────────────────┘             │
│                  ↕                             │
│        ┌─────────────────────────┐             │
│        │   CALCULATION MEANS     │  ⌐68        │
│        └─────────────────────────┘             │
│                  ↕                             │
│        ┌─────────────────────────┐             │
│        │    JUDGMENT MEANS       │  ⌐69        │
│        └─────────────────────────┘             │
└──────────────────────────────────────────────┘
```

# FIG.9

# FIG.10

### INTERNATIONAL SEARCH REPORT

| | International application No. |
|---|---|
| | PCT/JP2005/014303 |

| A. CLASSIFICATION OF SUBJECT MATTER |
|---|
| Int.Cl⁷ G01N27/62, 33/68 |

According to International Patent Classification (IPC) or to both national classification and IPC

| B. FIELDS SEARCHED |
|---|

Minimum documentation searched (classification system followed by classification symbols)
Int.Cl⁷ G01N27/62-27/70, H01J49/00-49/48

Documentation searched other than minimum documentation to the extent that such documents are included in the fields searched

| Jitsuyo Shinan Koho | 1922-1996 | Jitsuyo Shinan Toroku Koho | 1996-2005 |
|---|---|---|---|
| Kokai Jitsuyo Shinan Koho | 1971-2005 | Toroku Jitsuyo Shinan Koho | 1994-2005 |

Electronic data base consulted during the international search (name of data base and, where practicable, search terms used)
JSTPlus (JOIS)

| C. DOCUMENTS CONSIDERED TO BE RELEVANT |
|---|

| Category* | Citation of document, with indication, where appropriate, of the relevant passages | Relevant to claim No. |
|---|---|---|
| P,A | Tsuyoshi TABATA et al., "Proteomics Kaisekiyo Software no Kaihatsu - (1) Tanpakushitsu Hatsugen Teiryo Kaisekiyo Program -", Dai 27 Kai The Molecular Biology Society of Japan Nenkai Program Koen Yoshishu, 25 November, 2004 (25.11.04), page 811 | 1-10 |
| A | Yasuto YOKOI et al., "Focused Proteome ni Okeru Bioinformatics – 1: LC-MS/MS Data kara no Tanpakushitsu Dotei Sofuto no Hikaku· Hyoka", Dai 51 Kai Shitsuryo Bunseki Sogo Toronkai Koen Yoshishu, 14 May, 2003 (14.05.03) – 16 May, 2003 (16.05.03), pages 366 to 367 | 1-10 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 August, 2005 (29.08.05) | 20 September, 2005 (20.09.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

| | International application No. |
|---|---|
| | PCT/JP2005/014303 |

C (Continuation).   DOCUMENTS CONSIDERED TO BE RELEVANT

| Category* | Citation of document, with indication, where appropriate, of the relevant passages | Relevant to claim No. |
|---|---|---|
| A | Yasuto YOKOI et al., "Focused Proteome ni Okeru Bioinformatics – 2: Tanpakushitsu Dotei Kekka no Filtering Tool", Dai 51 Kai Shitsuryo Bunseki Sogo Toronkai Koen Yoshishu, 14 May, 2003 (14.05.03) – 16 May, 2003 (16.05.03), pages 368 to 369 | 1-10 |
| A | JP 2002-527756 A  (NeoGenesis Drug Discovery, Inc.), 27 August, 2002 (27.08.02) & WO 00/22649 A1 | 1-10 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. MANN ; P. HOJRUP ; P. ROEPSTORFF.** *Biol. Mass Spectrom.,* 1993, vol. 22, 338
- **J.K. ENG ; A.L. MCCORMACK ; I. YATES ; JOHN R.** *Journal of the American Society for Mass Spectrometry,* 1994, vol. 5, 976
- **M. MANN ; M. WILM.** *Anal. Chem.,* 1994, vol. 66, 4390
- **D.N. PERKINS ; D.J. PAPPIN ; D.M. CREASY ; J.S. COTTRELL.** *Electrophoresis,* 1999, vol. 20, 3551
- *Nat. Genet.,* 1998, vol. 20, 46-50
- *J. Cell Biol.,* 1998, vol. 141, 967-977
- *J. Cell Biol.,* 2000, vol. 148, 635-651
- *Nature,* 2002, vol. 415, 141-147
- *Nature,* 2002, vol. 415, 180-183
- *Curr. Opin. Cell Biol.,* 2003, vol. 15, 199-205
- *Curr. Opin. Cell Biol.,* 2003, vol. 7, 21-27
- **J. RAPPSILBER ; Y ISHIHAMA ; M. MANN.** *Anal. Chem.,* 2003, vol. 75, 663
- **Y. ISHIHAMA ; J. RAPPSILBER ; J.S. ANDERSEN ; M. MANN.** *J. Chromatogr. A,* 2002, vol. 979, 233